# EUROPÄISCHE PATENTSCHRIFT

(11) **EP 3 930 535 B1**
(45) Veröffentlichungstag und Bekanntmachung des Hinweises auf die Patenterteilung: **16.10.2024**
(21) Anmeldenummer: 20707084.8
(22) Anmeldetag: 25.02.2020
(51) Int. Cl.: A45F 5/02

(54) **MEDIZINISCHE HALTEVORRICHTUNG ZUR BEFESTIGUNG EINER MEDIZINISCHEN KITTELFLASCHE AN EINEM BEKLEIDUNGSSTÜCK**
MEDICAL HOLDING APPARATUS FOR SECURING A MEDICAL COAT POCKET BOTTLE TO A PIECE OF CLOTHING
SUPPORT MÉDICAL POUR LA FIXATION D'UN FLACON DE BLOUSE MÉDICAL SUR UN VÊTEMENT

(30) Priorität: 26.02.2019 DE 102019202574
(43) Veröffentlichungstag der Anmeldung: 05.01.2022
(73) Patentinhaber: B. Braun Melsungen AG, 34212 Melsungen (DE)
(72) Erfinder: HOFSTETTER, Rudolf, 6204 Sempach (CH)
(74) Vertreter: Patentanwälte Ruff, Wilhelm, Beier, Dauster & Partner mbB
(86) Internationale Anmeldenummer: PCT/EP2020/054857
(87) Internationale Veröffentlichungsnummer: WO 2020/173914

(56) Entgegenhaltungen:
- KR-U- 20100 003 524
- KR-U- 20100 003 524
- US-A- 5 765 888
- US-A- 5 765 888
- US-A- 5 890 635
- US-A- 5 890 635
- US-A1- 2005 109 803
- US-A1- 2005 109 803
- US-B1- 6 837 472
- US-B1- 6 837 472
- US-B1- 8 272 545
- US-B1- 8 272 545

## Beschreibung

Die Erfindung betrifft ein medizinisches System mit einer medizinischen Kittelflasche zur Aufnahme einer Flüssigkeit, einem an einem Halsabschnitt der Kittelflasche angeordneten und zur Entnahme der Flüssigkeit manuell betätigbaren Pumpspenderaufsatz und einer mit der Kittelflasche verbundenen medizinischen Haltevorrichtung. Die medizinische Haltevorrichtung ist zum Befestigen der medizinischen Kittelflasche an einem Bekleidungsstück eingerichtet und weist einen Ringabschnitt, der zur formschlüssigen Verbindung mit dem Halsabschnitt der Kittelflasche vorgesehen ist, und einen mit dem Ringabschnitt verbundenen Befestigungsabschnitt auf, der zur lösbaren Befestigung der mit der Kittelflasche verbundenen Haltevorrichtung an dem Bekleidungsstück vorgesehen ist.

Im medizinischen Bereich ist eine regelmäßige Händedesinfektion zur Vermeidung einer Übertragung von Krankheitserregern von einem Patienten über das Personal auf einen anderen Patienten gängige Praxis. Hierfür kommen üblicherweise stationär angeordnete und frei zugängliche Desinfektionsmittelspender zum Einsatz, aus denen das Personal bei Bedarf eine Desinfektionsflüssigkeit entnehmen kann.

Die Verwendung frei zugänglicher Desinfektionsmittelspender verbietet sich überall dort, wo dies mit einer Gefährdung für Patienten einhergehen könnte. Insbesondere in solchen Fällen ist die Verwendung sogenannter medizinischer Kittelflaschen zur Händedesinfektion üblich und allgemein bekannt. Solche Kittelflaschen sind am Körper tragbare Flüssigkeitskleingebinde mit einem nominellen Füllvolumen zwischen 25 ml und 150 ml. Kittelflaschen werden vom medizinischen Personal üblicherweise in einer Tasche eines Bekleidungsstücks, beispielsweise eines Kittels, mitgeführt und sind auf diese Weise bei Bedarf stets griffbereit.

Zur Händedesinfektion wird die Kittelflasche beispielsweise mit der rechten Hand aus der Tasche entnommen. Zur Entnahme von Desinfektionsflüssigkeit wird ein Klappdeckel der Kittelflasche mit dem Daumen derselben Hand geöffnet und Desinfektionsflüssigkeit durch Zusammenpressen und/oder Kippen der Kittelflasche in die hohle Handfläche der linken Hand gegossen. Hiernach wird der Klappdeckel mit dem Daumen der rechten Hand geschlossen und die Kittelflasche wird mit derselben Hand in der Tasche abgelegt. Erst hiernach wird die in der hohlen linken Handfläche befindliche Desinfektionsflüssigkeit beidhändig zwischen den Händen verrieben. All dies ist aufwändig und zeitintensiv, was zu einer Vernachlässigung der Händedesinfektion führen kann.

Um eine erleichterte Handhabung zu ermöglichen, ist eine Haltevorrichtung allgemein bekannt, die einem Befestigen der Kittelflasche an einem Bekleidungsstück dient. Zu diesem Zweck weist die Haltevorrichtung einen Ringabschnitt und einen mit dem Ringabschnitt verbundenen Befestigungsabschnitt auf. Der Ringabschnitt dient einer formschlüssigen Verbindung mit der Kittelflasche und wird hierfür auf einen Halsabschnitt der Kittelflasche gesteckt und durch Aufschrauben oder Aufstecken eines Verschlusses der Kittelflasche axial am Halsabschnitt fixiert. Der Befestigungsabschnitt dient einer lösbaren Befestigung der Haltevorrichtung und der mit dieser verbundenen Kittelflasche an dem Bekleidungsstück. Bei der bekannten Haltevorrichtung ist der Befestigungsabschnitt hierzu mit einer Klemme versehen, die üblicherweise als "Krokodil-Klemme" bezeichnet wird. Durch die Verwendung der bekannten Haltevorrichtung kann zwar auf ein Entnehmen und Ablegen der Kittelflasche aus bzw. in die Tasche verzichtet werden. Nachteilig ist jedoch, dass die Kittelflasche zur Entnahme der Desinfektionsflüssigkeit dennoch gegriffen, geöffnet, gedrückt und/oder gekippt und hiernach erneut geschlossen werden muss. Weiter nachteilig ist, dass die Kittelflasche hierbei mit einer potentiell verkeimten Hand gehandhabt und von dem Bekleidungsstück gelöst werden muss, wodurch Krankheitserreger auf die Kittelflasche übertragen werden können. Bei einer späteren erneuten Handhabung der Kittelflasche kann es daher zu einer Rückübertragung von Krankheitserregern auf das Personal kommen.

Aus der US 5 765 888 A, der KR 2010 0003524 U, der US 5 890 635 A und der US 2005/109803 A1 sind jeweils Anordnungen mit einer Flasche und einer Haltevorrichtung zum vereinfachten Tragen der Flasche bekannt. Die aus dem Stand der Technik bekannten Haltevorrichtungen sind jeweils an einem Halsabschnitt der Flasche befestigt oder befestigbar. Zudem sind die bekannten Haltevorrichtungen grundsätzlich zum Befestigen der Flasche geeignet, beispielsweise zum Befestigen der Flasche an einem Kleidungsstück,

Aufgabe der Erfindung ist es, ein medizinisches System der eingangs genannten Art bereitzustellen, das eine gegenüber dem Stand der Technik vereinfachte und in hygienischer Hinsicht verbesserte Handhabung bei einer Händedesinfektion ermöglicht.

Diese Aufgabe wird durch das Bereitstellen eines medizinischen Systems mit den Merkmalen des Anspruchs 1 gelöst. Bei dem erfindungsgemäßen medizinischen System ist wenigstens ein mit dem Ringabschnitt und dem Befestigungsabschnitt verbundener formstabiler Griffabschnitt vorgesehen, der in Radialrichtung des Ringabschnitts nach außen von dem Ringabschnitt abragt und eine Grifffläche aufweist, die bei einer manuellen in Axialrichtung des Ringabschnitts wirkenden Betätigung des an dem Halsabschnitt angeordneten Pumpspenderaufsatzes zur Entnahme von Flüssigkeit aus der Kittelflasche als axial wirkendes Gegenlager für wenigstens einen Finger einer den Pumpspenderaufsatz bedienenden Hand dient. Durch die erfindungsgemäße Lösung wird eine einhändige Entnahme von Flüssigkeit aus der Kittelflasche ermöglicht, ohne dass hierzu die Haltevorrichtung vom Bekleidungsstück gelöst werden muss. Hierdurch wird eine besonders hygienische Handhabung der Kittelflasche ermöglicht und gleichzeitig eine Zeitersparnis bei der Entnahme der Flüssigkeit erreicht. Der Griffabschnitt ragt in Radialrichtung des Ringabschnitts nach außen von demselben ab, so dass die Grifffläche - in einem mit der Kittelflasche verbundenen Zustand der Haltevorrichtung - vorzugsweise von einer Außenkontur der Kittelflasche abragt und manuell leicht erreichbar ist. Unter einer Kittelflasche ist im Rahmen dieser Erfindung ein am Körper tragbares medizinisches Flüssigkeitsgebinde zu verstehen. Die Kittelflasche kann insbesondere auch als Kleingebinde oder Pocket-Bottle bezeichnet werden. Die Kittelflasche weist vorzugsweise ein nominelles Füllvolumen von 25 ml bis 200 ml, bevorzugt zwischen 25 ml und 150 ml und besonders bevorzugt zwischen 40 ml und 100 ml auf. Kittelflaschen sind im medizinischen Bereich zur Verwendung bei der Händedesinfektion allgemein bekannt. Die Haltevorrichtung ist zur Verwendung mit einer mit einem manuell bedienbaren Pumpspenderaufsatz versehenen Kittelflasche vorgesehen. Pumpspenderaufsätze sind allgemein bekannt und dienen als Verschluss und Entnahmevorrichtung für Flüssigkeitsgebinde. Solche Pumpspenderaufsätze werden üblicherweise auf einen Halsabschnitt des Flüssigkeitsgebindes aufgeschraubt oder aufgesteckt und weisen ein in die zu entnehmende Flüssigkeit reichendes Steigrohr auf. Pumpspenderaufsätze können insbesondere auch als Pumpkappe oder Pumpverschluss bezeichnet werden. In einem auf die Kittelflasche aufgebrachten Zustand wird der Pumpspenderaufsatz zur Entnahme der Flüssigkeit in üblicher Weise in Axialrichtung der Kittelflasche wirkend manuell betätigt. Hierbei wird eine manuelle Druckkraft auf den Pumpspenderaufsatz aufgebracht. Die Druckkraft kann vorzugsweise mit dem Daumen einer Hand aufgebracht werden. Die Grifffläche bzw. der Griffabschnitt wirkt bei einer solchen Betätigung als Gegenlager und nimmt unter Anlage des wenigstens einen Fingers derselben Hand eine entgegen der Druckkraft wirkende Zugkraft auf. Anders ausgedrückt ist die medizinische Haltevorrichtung derart gestaltet, dass der Griffabschnitt - in einem mit der Kittelflasche verbundenen Zustand und bei einer Betätigung des Pumpspenderaufsatzes - ein Widerlager für eine Betätigungskraft des Pumpspenderaufsatzes bildet. Vorzugsweise umgreift der Ringabschnitt in verbundenem Zustand den Halsabschnitt in Umfangsrichtung vollständig. Der Befestigungsabschnitt kann zu einer mittelbaren oder unmittelbaren Befestigung an dem Bekleidungsstück vorgesehen sein.

In Ausgestaltung der Erfindung ist die Grifffläche gegenüber einer Axialebene des Ringabschnitts abgewinkelt. Demnach ist eine Normalenrichtung der Grifffläche nicht etwa parallel zu einer Axialrichtung des Ringabschnitts und/oder zu einer Betätigungsrichtung des Pumpspenderaufsatzes orientiert, sondern stattdessen schräg hierzu ausgerichtet. Hierdurch kann die Grifffläche in verbesserter Weise hintergriffen werden, wodurch ein Abgleiten des wenigstens einen Fingers von der Grifffläche bei der Betätigung des Pumpspenderaufsatzes vermieden werden kann.

In weiterer Ausgestaltung der Erfindung ist die Grifffläche in Axialrichtung des Ringabschnitts um einen Abstand von einer - in einem mit der Kittelflasche verbundenen Zustand - dem Pumpspenderaufsatz zugewandten Oberseite des Ringabschnitts beabstandet. Demnach ist die Grifffläche in Axialrichtung versetzt von der Oberseite angeordnet. Dieser Versatz erstreckt sich in Bezug auf eine übliche Orientierung der Kittelflasche mit vertikal nach oben ausgerichtetem Halsabschnitt vertikal nach unten. Der Abstand gewährleistet, dass die Grifffläche in ergonomischer Weise erreicht werden kann. Vorzugsweise ist der Abstand zwischen der Oberseite und der Grifffläche maßlich auf die zu befestigende Kittelflasche und den an dieser angeordneten Pumpspenderaufsatz angepasst. Der Abstand beträgt vorzugsweise 1 mm bis 100 mm, bevorzugt 5 mm bis 50 mm und besonders bevorzugt 10 mm bis 25 mm.

In weiterer Ausgestaltung der Erfindung sind der Befestigungsabschnitt und der Griffabschnitt in funktioneller Hinsicht gegeneinander austauschbar gleichartig gestaltet, wobei der Befestigungsabschnitt eine Grifffläche aufweist, die in Axialrichtung des Ringabschnitts von der Grifffläche des Griffabschnitts beabstandet angeordnet ist. In funktioneller Hinsicht gegeneinander austauschbar gleichartig gestaltet meint, dass der Befestigungsabschnitt alternativ als Griffabschnitt und der Griffabschnitt alternativ als Befestigungsabschnitt fungieren können. Da die Grifffläche des Befestigungsabschnitts in Axialrichtung des Ringabschnitts von der Grifffläche des Griffabschnitts beabstandet angeordnet ist, kann zur verbesserten ergonomischen Anpassung entschieden werden, welche der Griffflächen - nach der Verbindung der Haltevorrichtung mit der Kittelflasche - verwendet werden soll. In Abhängigkeit dieser Entscheidung kann die Haltevorrichtung auf die eine oder andere Art mit der Kittelflasche verbunden werden. Mit anderen Worten ausgedrückt ist bei dieser Ausgestaltung der Erfindung der Griffabschnitt zusätzlich zur lösbaren Befestigung der mit der Kittelflasche verbundenen Haltevorrichtung an dem Bekleidungsstück vorgesehen; der Befestigungsabschnitt ist hierbei formstabil gestaltet und die Grifffläche kann bedarfsweise in gleicher Weise wie die Grifffläche des Griffabschnitts als Gegenlager bei einer Betätigung des Pumpspenderaufsatzes dienen. Vorzugsweise sind der Befestigungsabschnitt und der Griffabschnitt in Umfangsrichtung des Ringabschnitts um 180° zueinander versetzt angeordnet. Je nachdem, welche der Griffflächen im verbundenen Zustand verwendet werden soll, wird die Haltevorrichtung dementsprechend in Umfangsrichtung ausgerichtet mit der Kittelflasche verbunden. Dies ist eine besonders vorteilhafte Ausgestaltung der Erfindung.

In weiterer Ausgestaltung der Erfindung weisen der Griffabschnitt und der Befestigungsabschnitt jeweils eine Befestigungsöffnung zur Verbindung mit einem Befestigungselement auf. Demnach ist bei dieser Ausgestaltung der Erfindung eine mittelbare Befestigung der Haltevorrichtung an dem Bekleidungsstück vorgesehen. Dabei dienen die Befestigungsöffnungen zur Verbindung mit einem Befestigungselement, das einends in und/oder an der Befestigungsöffnung und andernends an dem Bekleidungsstück befestigt werden kann. Als Befestigungselement kann insbesondere ein Clip, eine Klemme oder eine sogenannte Krokodil-Klemme verwendet werden.

Weiter gemäß der Erfindung weist der Ringabschnitt wenigstens ein Rastelement auf, das in Axialrichtung formschlüssig mit dem Halsabschnitt zusammenwirkt. Das wenigstens eine Rastelement dient demnach einer axialen Verbindung zwischen der Haltevorrichtung und der Kittelflasche. Das wenigstens eine Rastelement wirkt mit wenigstens einem am Halsabschnitt angeordneten Gegenrastelement zusammen. Diese Ausgestaltung der Erfindung ermöglicht eine besonders zuverlässige und anwendungssichere Verbindung zwischen der Haltevorrichtung und der Kittelflasche.

In weiterer Ausgestaltung der Erfindung weist der Ringabschnitt einen rohrförmig in Axialrichtung erstreckten Zylinderabschnitt mit einer Zylindermantelfläche auf, die - in einem mit der Kittelflasche verbundenen Zustand - radial an dem Halsabschnitt abgestützt ist. Die Zylindermantelfläche und der Halsabschnitt sind hierbei koaxial orientiert. Durch diese Ausgestaltung der Erfindung kann eine hinsichtlich der übertragbaren und abstützbaren Kräfte verbesserte formschlüssige Verbindung zwischen dem Ringabschnitt und dem Halsabschnitt erreicht werden. Die Zylindermantelfläche umgreift den Halsabschnitt in Umfangsrichtung und ist maßlich vorzugsweise derart auf die Maße des Halsabschnitts abgestimmt, dass sich eine Passung ergibt.

In weiterer Ausgestaltung der Erfindung ist der Zylinderabschnitt - in dem mit der Kittelflasche verbundenen Zustand - einends axial am Pumpspenderaufsatz und andernends axial an der Kittelflasche abgestützt. Demnach ist die Haltevorrichtung im verbundenen Zustand vereinfacht ausgedrückt in Axialrichtung zwischen dem Pumpspenderaufsatz und der Kittelflasche formschlüssig festgelegt, vorzugsweise geklemmt. Hierdurch kann eine nochmals verbesserte Verbindung zwischen der Haltevorrichtung und der Kittelflasche erreicht werden.

In weiterer Ausgestaltung der Erfindung ist eine einstückige Gestaltung der medizinischen Haltevorrichtung in Form eines aus Kunststoff gefertigten Spritzgussbauteils vorgesehen. Dies ermöglicht eine einfache und kostengünstige Fertigung.

In weiterer Ausgestaltung der Erfindung ist eine einstückige Gestaltung der medizinischen Haltevorrichtung in Form eines aus Metall gefertigten Bauteils vorgesehen. Gegenüber einer Fertigung aus Kunststoff kann hierdurch eine besonders formstabile und dennoch hinreichend dünnwandige Gestaltung der Haltevorrichtung erreicht werden.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivdarstellung eine medizinische Kittelflasche nach dem Stand der Technik, die mit einem Pumpspenderaufsatz nach dem Stand der Technik zur Entnahme einer Flüssigkeit aus der Kittelflasche versehen ist,
- Fig. 2: in schematischer Perspektivdarstellung eine aus dem Stand der Technik bekannte medizinische Haltevorrichtung, die zum Befestigen der Kittelflasche nach Fig. 1 an einem Bekleidungsstück vorgesehen ist,
- Fig. 3: in schematischer Perspektivdarstellung eine Ausführungsform einer erfindungsgemäßen medizinischen Haltevorrichtung,
- Fig. 4: in einer gegenüber der Ansicht nach Fig. 3 um in etwa 90° gedrehten Ansicht eine schematische Perspektivdarstellung einer weiteren Ausführungsform einer erfindungsgemäßen Haltevorrichtung und
- Fig. 5: in stark vereinfachter schematischer Perspektivdarstellung eine Ausführungsform eines erfindungsgemäßen medizinischen Systems.

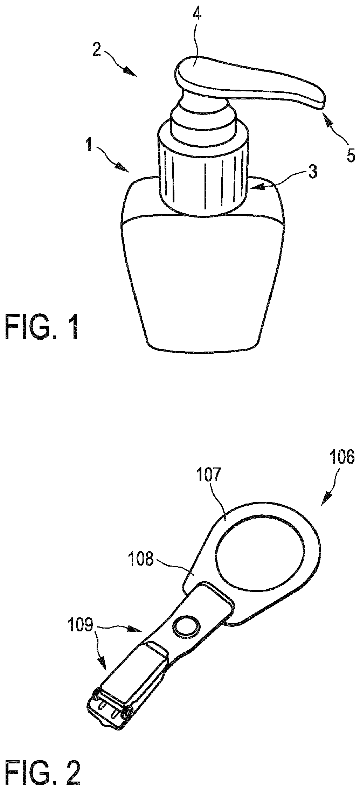

Gemäß Fig. 1 ist eine medizinische Kittelflasche 1 zur Verwendung bei einer Händedesinfektion vorgesehen und dementsprechend mit einer nicht näher bezeichneten Desinfektionsflüssigkeit befüllt. Ein nominelles Füllvolumen der Kittelflasche 1 beträgt vorliegend 100 ml und kann hiervon abweichend zwischen 25 ml und 200 ml betragen. Zur Entnahme der Desinfektionsflüssigkeit aus der Kittelflasche 1 ist diese vorliegend mit einem Pumpspenderaufsatz 2 versehen, der auf grundsätzlich bekannte Weise an einem Halsabschnitt 3 der Kittelflasche 1 festgelegt ist. Zur Entnahme der Desinfektionsflüssigkeit wird der Pumpspenderaufsatz 2 an einem oberseitig angeordneten Betätigungsabschnitt 4 manuell axial - in Bezug auf die Zeichenebene der Fig. 1 - nach unten gedrückt, wodurch im Ergebnis die Desinfektionsflüssigkeit durch einen im Detail nicht näher gezeigten Auslass 5 abgegeben wird. Der Aufbau und die Funktionsweise des Pumpspenderaufsatzes 2 sind grundsätzlich bekannt, so dass auf weitere Einzelheiten hierzu nicht weiter eingegangen werden muss. Die anhand Fig. 1 ersichtliche Kittelflasche 1 mitsamt dem daran befindlichen Pumpspenderaufsatz 2 ist dazu vorgesehen, von medizinischem Personal am Körper, beispielsweise in einer Tasche eines Bekleidungsstücks, mitgeführt zu werden. Hierdurch ist die Kittelflasche 1 stets griffbereit und eine Händedesinfektion kann bedarfsweise und ortsunabhängig erfolgen. Eine in der medizinischen Praxis übliche Art der Händedesinfektion kann vorsehen, dass die Kittelflasche 1 beispielsweise mit der rechten Hand gegriffen und aus der besagten Tasche entnommen wird. Hiernach kann der Pumpspenderaufsatz 2 mit dem Daumen derselben Hand zur Abgabe von Desinfektionsflüssigkeit in die hohle Handfläche der linken Hand betätigt werden. Die Kittelflasche 1 kann hiernach wieder in der Tasche abgelegt werden. Erst dann kann die in der linken Handfläche befindliche Desinfektionsflüssigkeit beidhändig zwischen den Händen verrieben werden.

Um eine im Vergleich hierzu vereinfachte Handhabung zu ermöglichen, ist im Stand der Technik eine anhand Fig. 2 ersichtliche medizinische Haltevorrichtung 106 bekannt. Mittels der Haltevorrichtung 106 kann die medizinische Kittelflasche 1 an einem Bekleidungsstück befestigt werden, so dass nicht zwingend eine Ablage in einer Tasche des Bekleidungsstücks notwendig ist. Hierzu weist die Haltevorrichtung 106 einen Ringabschnitt 107 und einen mit dem Ringabschnitt 107 verbundenen Befestigungsabschnitt 108 auf. Der Ringabschnitt 107 ist zur formschlüssigen Verbindung mit dem Halsabschnitt 3 vorgesehen. Zur Verbindung kann der Pumpspenderaufsatz 2 ausgehend von der anhand Fig. 1 ersichtlichen Konfiguration zunächst vom Halsabschnitt 3 gelöst werden. In diesem Zustand kann der Ringabschnitt 107 axial über den Halsabschnitt 3 geführt werden, so dass eine formschlüssige Verbindung in radialer Richtung erreicht ist. Durch erneutes Aufbringen des Pumpspenderaufsatzes 2 auf den nun von dem Ringabschnitt 107 umgriffenen Halsabschnitt 3 wird der Ringabschnitt 107 auch in axialer Richtung formschlüssig festgelegt. Der Befestigungsabschnitt 108 ist zur lösbaren Befestigung an dem besagten Kleidungsstück, beispielsweise einem Kittel oder einem Gürtel, vorgesehen und weist hierzu eine nicht näher bezeichnete Befestigungsöffnung auf, die mit einem üblicherweise als Krokodil-Klemme bezeichneten Befestigungselement 109 zusammenwirkt. Die Krokodil-Klemme 109 kann auf grundsätzlich bekannte Weise an dem Kleidungsstück befestigt werden. Durch die Verwendung der bekannten Haltevorrichtung 106 kann die Kittelflasche 1 zwar außenliegend an dem Bekleidungsstück mitgeführt werden, so dass auf ein Ablegen in und ein Entfernen aus der Tasche verzichtet werden kann. Dennoch muss die Haltevorrichtung 106 zur Entnahme von Desinfektionsflüssigkeit üblicherweise zunächst von dem Bekleidungsstück gelöst werden, da ansonsten allenfalls eine ergonomisch sehr unvorteilhafte Betätigung des Pumpspenderaufsatzes 2 möglich ist.

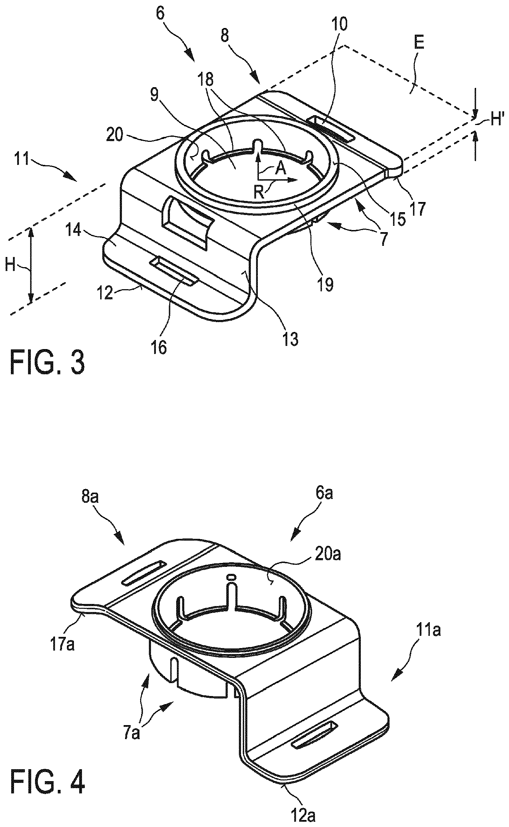

Um eine weiter vereinfachte und insbesondere in hygienischer Hinsicht verbesserte Handhabung der Kittelflasche 1 bei der Händedesinfektion zu ermöglichen, ist eine erfindungsgemäße medizinische Haltevorrichtung 6 gemäß Fig. 3 vorgesehen. Die Haltevorrichtung 6 weist einen Ringabschnitt 7 und einen mit dem Ringabschnitt 7 verbundenen Befestigungsabschnitt 8 auf. Der Ringabschnitt 7 umgrenzt eine zentrale Durchgangsöffnung 9, die vorliegend einen kreisförmigen Querschnitt aufweist. Der Ringabschnitt 7 dient einer formschlüssigen Verbindung mit dem Halsabschnitt 3 der Kittelflasche 1. Hierzu wird die Haltevorrichtung 6 in einem nicht mit dem Pumpspenderaufsatz 2 versehenen Zustand der Kittelflasche 1 entlang einer Axialrichtung A des Ringabschnitts 7 mit der Durchgangsöffnung 9 über den Halsabschnitt 3 geführt. Hierdurch ergibt sich eine in Radialrichtung R des Ringabschnitts 7 formschlüssige Verbindung zwischen der Haltevorrichtung 6 und der Kittelflasche 1. Wird der Pumpspenderaufsatz 2 hiernach auf grundsätzlich bekannte Weise mit dem Halsabschnitt 3 verbunden, ist die Haltevorrichtung 6 zudem in Axialrichtung A formschlüssig zwischen der Kittelflasche 1 und dem Pumpspenderaufsatz 2 festgelegt.

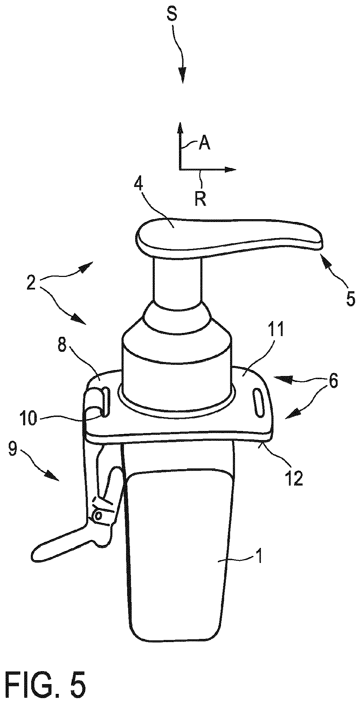

Der Befestigungsabschnitt 8 grenzt vorliegend in Radialrichtung R an eine nicht näher bezeichnete Außenkontur der Durchgangsöffnung 9 an und ist zur wenigstens mittelbaren lösbaren Befestigung an einem Bekleidungsstück vorgesehen. Vorliegend weist der Befestigungsabschnitt 8 hierzu eine Befestigungsöffnung 10 auf, die in Axialrichtung A schlitzförmig durch den Befestigungsabschnitt 8 erstreckt und zur Verbindung mit einem Befestigungselement 9 (vgl. Fig. 5) vorgesehen ist. Das Befestigungselement 9 kann beispielsweise in Form einer Krokodil-Klemme 109 (vgl. Fig. 2) ausgebildet sein und einen grundsätzlich bekannten Aufbau aufweisen.

Im Unterschied zu der bekannten Haltevorrichtung 106 weist die erfindungsgemäße Haltevorrichtung 6 einen formstabilen Griffabschnitt 11 auf. Der Griffabschnitt 11 ist mit dem Ringabschnitt 7 und - jedenfalls mittelbar über den Ringabschnitt 7 - mit dem Befestigungsabschnitt 8 verbunden. Der Griffabschnitt 11 ragt in Radialrichtung R des Ringabschnitts 7 nach außen von dem Ringabschnitt 7 ab und weist eine Grifffläche 12 auf. Die Grifffläche 12 ist - in Bezug auf die Zeichenebene der Fig. 3 - unterseitig an dem Griffabschnitt 11 angeordnet und vorliegend in etwa horizontal erstreckt. Eine nicht näher bezeichnete Normalenrichtung der Grifffläche 12 ist somit vorliegend parallel zu der anhand Fig. 3 ersichtlichen Axialrichtung A orientiert.

Anhand Fig. 5 ist in schematisch stark vereinfachter Weise ein medizinisches System S ersichtlich, das durch ein Verbinden der Haltevorrichtung 6 mit der Kittelflasche 1 nebst Pumpspenderaufsatz 2 gebildet ist. Dort ist erkennbar, dass der Griffabschnitt 11 über eine nicht näher bezeichnete Außenkontur der Kittelflasche 1 ragt und eine Art Vorsprung bildet. In der anhand Fig. 5 ersichtlichen Konfiguration sind der Auslass 5 und der Griffabschnitt 11 in Radialrichtung R miteinander fluchtend ausgerichtet. Dies ist insbesondere aufgrund der üblicherweise vorgesehenen Schwenkbeweglichkeit des Auslasses 5 in Umfangsrichtung ohne weiteres möglich.

Zur Entnahme von Desinfektionsflüssigkeit ausgehend von der anhand Fig. 5 ersichtlichen Konfiguration wird der Betätigungsabschnitt 4 mit dem Daumen einer Hand entgegen der in Fig. 5 eingezeichneten Axialrichtung A nach unten gedrückt. Hierbei umgreift die Handfläche derselben Hand den Auslass 5 in Radialrichtung außenseitig und wenigstens ein Finger derselben Hand hintergreift die Grifffläche 12 in Axialrichtung A von unten nach oben gerichtet. Auf diese Weise wirkt der Griffabschnitt 11 als Gegenlager bei der Betätigung des Pumpspenderaufsatzes 2, wobei der Griffabschnitt 11 eine von dem wenigstens einen Finger an der Grifffläche 12 eingeleitete in Axialrichtung A wirkende Gegenkraft zur Betätigung des Betätigungsabschnitts 4 aufnimmt. Mit andern Worten ausgedrückt fungiert der Griffabschnitt 11 hierbei als Widerlager. Bei dieser Art der Handhabung wird die Desinfektionsflüssigkeit durch den Auslass 5 in die Handfläche und/oder auf die Innenseite der Finger der den Pumpspenderaufsatz 2 betätigenden Hand abgegeben. Dies ermöglicht eine einhändige Entnahme von Desinfektionsflüssigkeit, ohne dass hierfür das System S von dem Bekleidungsstück gelöst oder von der verbleibenden Hand gegriffen werden muss.

Wie weiter anhand Fig. 3 ersichtlich ist, ist der Griffabschnitt 11 vorliegend gegenüber einer Axialebene E des Ringabschnitts 7 abgestuft ausgebildet. Die Axialebene E ist vorliegend in Bezug auf die Axialrichtung A in etwa mittig durch den Ringabschnitt 7 erstreckt und insoweit eine Mittelebene. Die Axialebene E ist in dem anhand Fig. 5 ersichtlichen Zustand senkrecht zur Betätigungsrichtung des Pumpspenderaufsatzes 2 orientiert. Durch die abgestufte Ausbildung des Griffabschnitts weist dieser vorliegend einen parallel zur Axialrichtung A erstreckten ersten Wandabschnitt 13 und einen in Radialrichtung R erstreckten zweiten Wandabschnitt 14 auf. Die Grifffläche 12 ist vorliegend unterseitig an dem zweiten Wandabschnitt 14 angeordnet.

Bei der vorliegenden Gestaltung ist die Grifffläche 12 in Axialrichtung A um einen Abstand H von einer - in dem anhand Fig. 5 ersichtlichen Zustand - dem Pumpspenderaufsatz 2 zugewandten Oberseite 15 des Ringabschnitts 7 beabstandet. Der Abstand H gewährleistet eine besonders ergonomische Handhabung der Grifffläche 12 und kann insbesondere auf die axiale Bauhöhe des Pumpspenderaufsatzes 2 (vgl. Fig. 5) oder eine Fingerlänge oder eine Handgröße abgestimmt sein.

Anhand Fig. 3 ist zudem ersichtlich, dass vorliegend auch der Griffabschnitt 12 mit einer Befestigungsöffnung 16 versehen ist. Die Befestigungsöffnung 16 ist gleichartig zu der Befestigungsöffnung 10 ausgebildet und dient einer bedarfsweisen Verbindung mit dem Befestigungselement 9. Insoweit sind der Griffabschnitt 11 und der Befestigungsabschnitt 8 im Hinblick auf die Befestigungsöffnungen 10, 16 gleichartig gestaltet. Außerdem ist der Befestigungsabschnitt 8 vorliegend gegenüber der Axialebene E geringfügig nach unten abgewinkelt und weist unterseitig eine Grifffläche 17 auf. Demnach ist der Befestigungsabschnitt 8 im Hinblick auf das Vorhandensein einer Grifffläche gleichartig zu dem Griffabschnitt 11 gestaltet. Die Grifffläche 17 ist in Axialrichtung A um einen Abstand H` von der Axialebene E beabstandet. Der Abstand H` ist vorliegend deutlich geringer als der Abstand H. Weiterhin ragt der Befestigungsabschnitt in Radialrichtung R von dem Ringabschnitt 7 nach außen ab. Im Ergebnis sind der Befestigungsabschnitt 8 und der Griffabschnitt 11 vorliegend in funktioneller Hinsicht gegeneinander austauschbar gleichartig gestaltet. Das heißt bedarfsweise kann die Haltevorrichtung 6 im Hinblick auf die Positionierung des Griffabschnitts 11 und des Befestigungsabschnitts 8 unterschiedlich mit der Kittelflasche 1 verbunden werden. Eine Möglichkeit sieht vor, dass - wie vorbeschrieben - der Griffabschnitt 11 in Axialrichtung A unterhalb des Auslasses 5 und in Radialrichtung R fluchtend mit diesem positioniert wird. Eine weitere Möglichkeit sieht vor, dass stattdessen der Befestigungsabschnitt 8 an dieser Stelle positioniert wird, wozu die Haltevorrichtung 6 vorliegend um 180° in Umfangsrichtung gedreht anzubringen ist. Auf diese Weise gewährleistet die Haltevorrichtung 6 eine besonders einfache und anforderungsgerechte Anpassung des jeweils erforderlichen axialen Abstands zwischen der Grifffläche 12, 17 und dem Pumpspenderaufsatz 2 bzw. des Abstands H, H'. Hierdurch kann das medizinische System S durch eine entsprechende Positionierung der Haltevorrichtung 6 gegenüber dem Auslass 5 an eine Handgröße oder eine Fingerlänge oder die Bauhöhe des Pumpspenderaufsatzes 2 angepasst werden.

Der Ringabschnitt 7 weist vorliegend mehrere in Umfangsrichtung zueinander beabstandet angeordnete Rastelemente 18 auf, die in dem anhand Fig. 5 ersichtlichen Zustand auf zeichnerisch nicht dargestellte Weise in Axialrichtung A formschlüssig mit entsprechenden Gegenrastelementen des Halsabschnitts 3 zusammenwirken. Hierbei hintergreifen die Rastelemente 18 den Halsabschnitt 3 in Radialrichtung R lösbar federelastisch.

Weiterhin weist der Ringabschnitt 7 vorliegend einen in Axialrichtung A rohrförmig erstreckten Zylinderabschnitt 19 mit einer Zylindermantelfläche 20 auf. Der Zylinderabschnitt 19 ist in Axialrichtung A durch die vorliegend im Übrigen plattenförmig gestalteten Abschnitte der Haltevorrichtung 6 erstreckt und formstabil ausgebildet. Die Zylindermantelfläche 20 weist eine kreiszylindrische Kontur auf und ist in dem anhand Fig. 5 ersichtlichen mit dem Halsabschnitt 3 verbundenen Zustand radial an demselben abgestützt. Durch diese Gestaltung kann die Haltevorrichtung 6 insbesondere bei einer Krafteinwirkung auf die Grifffläche 12, 17 kippsicher an der Kittelflasche 1 abgestützt werden.

In mit der Kittelflasche 1 zusammengefügtem Zustand ist der Zylinderabschnitt 19 einends axial am Pumpspenderaufsatz 2 und andernends an einem nicht näher bezeichneten Abschnitt der Kittelflasche 1 abgestützt. Anders ausgedrückt ist die Haltevorrichtung 6 auf diese Weise zwischen dem Pumpspenderaufsatz 2 und der Kittelflasche 1 in Axialrichtung A eingeklemmt. Die Haltevorrichtung 6 ist vorliegend in Form eines einstückig gestalteten Spritzgussbauteils aus Kunststoff gefertigt. Dabei weist die Haltevorrichtung 6 vorliegend in einer axial von oben gerichteten Blickrichtung eine rechteckige Außenkontur auf, was jedoch nicht zwingend ist.

Die Ausführungsform nach Fig. 4 entspricht im Wesentlichen der zuvor anhand der Fig. 3 und 5 beschriebenen Haltevorrichtung 6. Zur Vermeidung von Wiederholungen wird daher auf die Offenbarung zu dieser Ausführungsform verwiesen. Identische Bauteile und Abschnitte werden bei der Ausführungsform nach Fig. 4 insoweit nicht gesondert erläutert. Funktionsgleiche Bauteile und Abschnitte, die jedoch in ihrer konstruktiven Ausführung unterschiedlich sind, sind mit gleichen Bezugszeichenziffern unter Hinzufügung des Kleinbuchstabens a bezeichnet.

Die Haltevorrichtung 6a unterscheidet sich im Wesentlichen dahingehend von der Haltevorrichtung 6, dass diese aus Metall gefertigt ist. Zudem sind einzelne Abmessungen unterschiedlich gewählt. Insbesondere der Zylinderabschnitt 20a ist vergleichsweise länger, zudem sind die Griffflächen 12a, 17a unterschiedlich beabstandet. Mit der Ausführungsform nach Fig. 4 ist verdeutlicht, dass die erfindungsgemäße Haltevorrichtung auf einfache Weise an unterschiedliche Gebindegrößen der Kittelflasche 1 und/oder Abmessungen des Pumpspenderaufsatzes 2 anpassbar ist.

## Patentansprüche

1. Medizinisches System (S) mit
einer medizinischen Kittelflasche (1) zur Aufnahme einer Flüssigkeit,
einem Pumpspenderaufsatz (2), der an einem Halsabschnitt (3) der Kittelflasche (1) angeordnet und zur Entnahme der Flüssigkeit manuell betätigbar ist, und
einer Haltevorrichtung (6, 6a), die mit der Kittelflasche (1) verbunden und zum Befestigen der medizinischen Kittelflasche (1) an einem Bekleidungsstück eingerichtet ist,
wobei die Haltevorrichtung (6, 6a) einen Ringabschnitt (7, 7a), der zur formschlüssigen Verbindung mit dem Halsabschnitt (3) der Kittelflasche (1) vorgesehen ist, und einen mit dem Ringabschnitt (7, 7a) verbundenen Befestigungsabschnitt (8, 8a), der zur lösbaren Befestigung der mit der Kittelflasche (1) verbundenen Haltevorrichtung (6, 6a) an dem Bekleidungsstück vorgesehen ist, aufweist,
wobei wenigstens ein mit dem Ringabschnitt (7, 7a) und dem Befestigungsabschnitt (8, 8a) verbundener formstabiler Griffabschnitt (11) vorgesehen ist, der in Radialrichtung (R) des Ringabschnitts nach außen von dem Ringabschnitt (7, 7a) abragt und eine Grifffläche (12, 12a) aufweist, die bei einer manuellen in Axialrichtung (A) des Ringabschnitts (7, 7a) wirkenden Betätigung des an dem Halsabschnitt (3) angeordneten Pumpspenderaufsatzes (2) zur Entnahme von Flüssigkeit aus der Kittelflasche (1) als axial wirkendes Gegenlager für wenigstens einen Finger einer den Pumpspenderaufsatz (2) bedienenden Hand dient,
und wobei der Ringabschnitt (7, 7a) wenigstens ein Rastelement (18) aufweist, das in Axialrichtung (A) formschlüssig mit einem an dem Halsabschnitt (3) angeordneten Gegenrastelement zusammenwirkt.

2. Medizinisches System (S) nach Anspruch 1, **dadurch gekennzeichnet, dass** die Grifffläche (12, 12a) gegenüber einer Axialebene (E) des Ringabschnitts (7, 7a) abgewinkelt ist.

3. Medizinisches System (S) nach Anspruch 1 oder 2, **dadurch gekennzeichnet, dass** die Grifffläche (12, 12a) in Axialrichtung (A) des Ringabschnitts (7, 7a) um einen Abstand (H) von einer dem Pumpspenderaufsatz (2) zugewandten Oberseite (15) des Ringabschnitts (7, 7a) beabstandet ist.

4. Medizinisches System (S) nach einem der vorhergehenden Ansprüche, **dadurch gekennzeichnet, dass** der Befestigungsabschnitt (8, 8a) und der Griffabschnitt (11) in funktioneller Hinsicht gegeneinander austauschbar gleichartig gestaltet sind, wobei der Befestigungsabschnitt (8, 8a) eine Grifffläche (17, 17a) aufweist, die in Axialrichtung (A) des Ringabschnitts (7, 7a) von der Grifffläche (12, 12a) des Griffabschnitts (11) beabstandet angeordnet ist.

5. Medizinisches System (S) nach Anspruch 4, **dadurch gekennzeichnet, dass** der Griffabschnitt (11) und der Befestigungsabschnitt (8, 8a) jeweils eine Befestigungsöffnung (16, 10) zur Verbindung mit einem Befestigungselement (9) aufweisen.

6. Medizinisches System (S) nach einem der vorhergehenden Ansprüche, **dadurch gekennzeichnet, dass** der Ringabschnitt (7, 7a) einen rohrförmig axial erstreckten Zylinderabschnitt (19) mit einer Zylindermantelfläche (20, 20a) aufweist, die radial an dem Halsabschnitt (3) abgestützt ist.

7. Medizinisches System (S) nach Anspruch 6, **dadurch gekennzeichnet, dass** der Zylinderabschnitt (19) einends axial am Pumpspenderaufsatz (2) und andernends axial an der Kittelflasche (1) abgestützt ist.

8. Medizinisches System (S) nach einem der vorhergehenden Ansprüche, wobei die Haltevorrichtung eine einstückige Gestaltung in Form eines aus Kunststoff gefertigten Spritzgussbauteils (6) aufweist.

9. Medizinisches System (S) nach einem der Ansprüche 1 bis 7, wobei die Haltevorrichtung eine einstückige Gestaltung in Form eines aus Metall gefertigten Bauteils (6a) aufweist.

## Claims

1. Medical system (S) with
a medical gown bottle (1) for holding a liquid,
a pump dispenser attachment (2), which is arranged on a neck portion (3) of the gown bottle (1) and can be actuated manually to dispense the liquid, and
a holding device (6, 6a) which is connected to the gown bottle (1) and is configured to attach the medical gown bottle (1) to an item of clothing,
wherein the holding device (6, 6a) has a ring portion (7, 7a), which is provided for positive connection to the neck portion (3) of the gown bottle (1), and a fastening portion (8, 8a), which is connected to the ring portion (7, 7a) and is provided for releasable fastening of the holding device (6, 6a) connected to the gown bottle (1) to the item of clothing,
wherein at least one dimensionally stable handle portion (11) connected to the ring portion (7, 7a) and the fastening portion (8, 8a) is provided, which projects outwards from the ring portion (7, 7a) in the radial direction (R) of the ring portion and has a gripping surface (12, 12a), which, when the pump dispenser attachment (2) arranged on the neck portion (3) is actuated manually in the axial direction (A) of the ring portion (7, 7a) for dispensing liquid from the gown bottle (1), serves as an axially acting counter-bearing for at least one finger of a hand operating the pump dispenser attachment (2),
and wherein the ring portion (7, 7a) has at least one latching element (18) which interacts positively in the axial direction (A) with a counter-latching element arranged on the neck portion (3).

2. Medical system (S) according to claim 1, **characterized in that** the gripping surface (12, 12a) is angled with respect to an axial plane (E) of the ring portion (7, 7a).

3. Medical system (S) according to claim 1 or 2, **characterized in that** the gripping surface (12, 12a) is spaced apart in the axial direction (A) of the ring portion (7, 7a) by a distance (H) from an upper side (15) of the ring portion (7, 7a) facing the pump dispenser attachment (2).

4. Medical system (S) according to any one of the preceding claims, **characterized in that** the fastening portion (8, 8a) and the handle portion (11) are designed to be interchangeably similar in functional terms, the fastening portion (8, 8a) having a gripping surface (17, 17a) which is arranged at a distance from the gripping surface (12, 12a) of the handle portion (11) in the axial direction (A) of the ring portion (7, 7a).

5. Medical system (S) according to claim 4, **characterized in that** the handle portion (11) and the fastening portion (8, 8a) each have a fastening opening (16, 10) for connection to a fastening element (9).

6. Medical system (S) according to any one of the preceding claims, **characterized in that** the ring portion (7, 7a) has a tubular axially extending cylindrical portion (19) with a cylindrical lateral surface (20, 20a) which is radially supported on the neck portion (3).

7. Medical system (S) according to claim 6, **characterized in that** the cylinder portion (19) is supported at one end axially on the pump dispenser attachment (2) and at the other end axially on the gown bottle (1).

8. Medical system (S) according to any one of the preceding claims, wherein the holding device has a one-piece design in the form of an injection-molded component (6) made of plastic.

9. Medical system (S) according to any one of claims 1 to 7, wherein the holding device has a one-piece design in the form of a component (6a) made of metal.

## Revendications

1. Système médical (S) comprenant
un flacon de blouse médicale (1) destiné à contenir un liquide,
un embout distributeur de pompe (2) disposé sur une partie de col (3) du flacon de blouse (1) et pouvant être actionné manuellement pour prélever le liquide, et
un dispositif de retenue (6, 6a) qui est relié au flacon de blouse (1) et qui est conçu pour fixer le flacon de blouse (1) à un vêtement,
le dispositif de retenue (6, 6a) présentant une section annulaire (7, 7a) qui est prévue pour la liaison par complémentarité de forme avec la section de col (3) du flacon de blouse (1), et une section de fixation (8, 8a) reliée à la section annulaire (7, 7a), qui est prévue pour la fixation amovible du dispositif de retenue (6, 6a) relié au flacon de blouse (1) sur la pièce de vêtement,
au moins une section de préhension (11) de forme stable, reliée à la section annulaire (7, 7a) et à la section de fixation (8, 8a), étant prévue, laquelle fait saillie vers l'extérieur de la section annulaire (7, 7a) dans la direction radiale (R) de la section annulaire et présente une surface de préhension (12, 12a), qui, lors d'un actionnement manuel agissant dans la direction axiale (A) du tronçon annulaire (7, 7a) de l'embout distributeur à pompe (2) disposé sur le tronçon de col (3) pour prélever du liquide de la blouse (1), sert de contre-appui agissant axialement pour au moins un doigt d'une main manipulant l'embout distributeur à pompe (2),
et la section annulaire (7, 7a) présentant au moins un élément d'encliquetage (18) qui, dans la direction axiale (A), coopère par complémentarité de forme avec un contre-élément d'encliquetage disposé sur la section de col (3).

2. Système médical (S) selon la revendication 1, **caractérisé en ce que** la surface de préhension (12, 12a) est coudée par rapport à un plan axial (E) de la partie annulaire (7, 7a).

3. Système médical (S) selon la revendication 1 ou 2, **caractérisé en ce que** la surface de préhension (12, 12a) est espacée dans la direction axiale (A) de la section annulaire (7, 7a) d'une distance (H) d'une face supérieure (15) de la section annulaire (7, 7a) tournée vers l'embout distributeur de pompe (2).

4. Système médical (S) selon l'une des revendications précédentes, **caractérisé en ce que** la section de fixation (8, 8a) et la section de préhension (11) sont conçues de manière identique et interchangeable d'un point de vue fonctionnel, la section de fixation (8, 8a) présentant une surface de préhension (17, 17a) qui est disposée à distance de la surface de préhension (12, 12a) de la section de préhension (11) dans la direction axiale (A) de la section annulaire (7, 7a).

5. Système médical (S) selon la revendication 4, **caractérisé en ce que** la partie de poignée (11) et la partie de fixation (8, 8a) présentent chacune une ouverture de fixation (16, 10) pour la liaison avec un élément de fixation (9).

6. Système médical (S) selon l'une des revendications précédentes, **caractérisé en ce que** la section annulaire (7, 7a) comprend une section cylindrique (19) s'étendant axialement de manière tubulaire avec une surface d'enveloppe cylindrique (20, 20a) qui est supportée radialement sur la section de col (3).

7. Système médical (S) selon la revendication 6, **caractérisé en ce que** la section de cylindre (19) est supportée axialement à une extrémité sur l'embout distributeur de pompe (2) et à l'autre extrémité axialement sur le flacon de blouse (1).

8. Système médical (S) selon l'une des revendications précédentes, dans lequel le dispositif de maintien présente une conception monobloc sous la forme d'un composant moulé par injection (6) réalisé en matière plastique.

9. Système médical (S) selon l'une des revendications 1 à 7, dans lequel le dispositif de maintien présente une configuration monobloc sous la forme d'un composant (6a) réalisé en métal.
